(19) Europäisches Patentamt
European Patent Office
Office européen des brevets

(11) Veröffentlichungsnummer: **0 261 668**
**B1**

# (12) EUROPÄISCHE PATENTSCHRIFT

(45) Veröffentlichungstag der Patentschrift: **02.05.90**

(51) Int. Cl.⁴: **C07D 333/32,** C07C 327/00

(21) Anmeldenummer: **87113926.7**

(22) Anmeldetag: **23.09.87**

(54) **Verfahren zur Herstellung von 4-Alkoxy-2(5H)-thiophenonen.**

(30) Priorität: **24.09.86 CH 3827/86**

(43) Veröffentlichungstag der Anmeldung:
**30.03.88 Patentblatt 88/13**

(45) Bekanntmachung des Hinweises auf die Patenterteilung:
**02.05.90 Patentblatt 90/18**

(84) Benannte Vertragsstaaten:
**AT BE CH DE ES FR GB IT LI LU NL SE**

(56) Entgegenhaltungen:
**EP-A- 0 189 096**
**EP-A- 0 189 097**

(73) Patentinhaber: **LONZA AG, Gampel/Wallis(CH)**

(72) Erfinder: **Meul, Thomas, Dr., Balfrinstrasse 21, Visp Wallis(CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein neues Verfahren zur Herstellung von 4-Alkoxy-2(5H)-thiophenonen.

Diese Verbindungen bilden wertvolle Zwischenprodukte zur Herstellung von Thiotetronsäurederivaten, insbesondere von hochreiner Thiotetronsäure.

Thiotetronsäure könnte als Zwischenprodukt zur Herstellung von (±)-Thiolactomycin, einem Antibiotikum mit breitem Wirkungsspektrum, eine bedeutende Anwendungsmöglichkeit finden (Tetrahedron Letters, Vol.25, Nr.46 pp, S.5243-5246, 1984).

Bisher hat es an vorteilhaften Verfahren gemangelt, Thiotetronsäure, insbesondere Thiotetronsäure in hochreiner Form, in guter Ausbeute herzustellen.

Aus E.Benary, Chem. Berichte 46, 2103 (1913) ist bekannt, die Thiotetronsäure, ausgehend von Acetylthioglycoylchlorid, durch Reaktion mit Natriummalonester und anschliessendem Ringschluss und Wasserbehandlung herzustellen.

D.B.Macierewicz, Rocz.Chem. 47, 1735 (1973) hat die Reaktion von E.Benary nachvollzogen und dabei Thiotetronsäure in einer Ausbeute von 30,3%, bezogen auf das eingesetzte Acetylthioglycoylchlorid, erhal ten.

Eine andere Möglichkeit zeigt die Synthese von J.Z.Mortensen et al, Tetrahedron 27, 3839 (1971). Ausgehend von 2,4-Dibromthiophen erhält er über 3 Stufen durch Umsetzung mit Butyllithium und t-Butylperbenzoat in einer Ausbeute von 46,2% die Thiotetronsäure.

Aus der EP-Anmeldung 0 189 097 ist ausserdem bekannt, Thiotetronsäure durch Umsetzung von Chloracetessigsäurechlorid mit $H_2S$ in Gegenwart von Trimethylamin herzustellen. Nachteilig erweist sich bei diesem Verfahren, dass die Thiotetronsäure nur über eine aufwendige Extraktion mit unzulänglicher Qualität (Gehalt 88%) hergestellt werden kann. Zudem ist das Arbeiten mit gasförmigem $H_2S$ im Hinblick auf ein technisches Verfahren nicht unproblematisch.

Zur Herstellung einer besseren Thiotetronsäurequalität ist aus der EP-Anmeldung 0 189 096 ein Verfahren bekannt, das sich auszeichnet durch die Umsetzung von 4-Chlor-4-chlormethyloxetan-2-on mit Schwefelwasserstoff in Gegenwart eines Amins direkt zur Thiotetronsäure, oder in einer zweiten Variante durch die Umsetzung der nicht isolierten Thiotetronsäure mit Keten zum 2,4-Diacetoxythiophen, das seinerseits mit einer Mineralsäure zur Thiotetronsäure umgesetzt wird. Nachteilig erweist sich bei diesem Verfahren, dass von einem Edukt ausgegangen werden muss, das nicht grosstechnisch verfügbar ist, sondern in einem separaten Syntheseschritt hergestellt werden muss. Zudem ist eine reine Thiotetronsäure gemäss der ersten Variante nur nach einer chromatographischen Reinigung und gemäss der zweiten Variante nur über den Umweg der Herstellung des leicht zu reinigenden 2,4-Diacetoxythiophens möglich. Auch bei diesem Verfahren muss wegen der Anwendung von $H_2S$ die Durchführung im technischen Rahmen als nicht unproblematisch gewertet werden.

Es bestand daher die Aufgabe einen Weg zu finden, der nicht mit diesen Nachteilen behaftet ist.

Ueberraschenderweise wurde ein technisch machbares Verfahren gefunden, das es zulässt, ohne die Verwendung des problematischen Schwefelwasserstoffes, ausgehend von einem grosstechnisch verfügbaren Halogenacetessigsäurealkylester durch Umsetzung mit Orthoameisensäuretrialkylester und Weiterumsetzung des 3-Alkoxy-4-halogen-2E-butensäurealkylesters gemäss Anspruch 1 4-Alkoxy-2(5H)-thiophenone der Formel

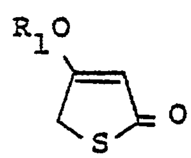

1

worin $R_1$ Alkyl geradkettig oder verzweigt, mit 1 bis 4 C-Atomen bedeutet, herzustellen, die interessante Ausgangsprodukte für weitere Thiotetronsäurederivate darstellen oder in hervorragender Weise als Zwischenstufe für hochreine Thiotetronsäure geeignet sind.

Zweckmässig wird so vorgegangen, dass in einer ersten Stufe auf bekannte Weise, z.B. gemäss CH-Patentgesuch 4119/85, aus 4-Halogen-acetessigsäurealkylester durch Umsetzung mit einem Orthoameisensäuretrialkylester in Gegenwart einer Säure ein 3-Alkoxy-4-halogen-2E-butensäurealkylester der Formel

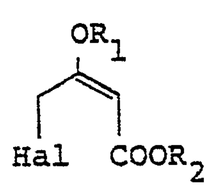

2

worin $R_1$ und $R_2$ Alkyl geradkettig oder verzweigt, mit 1 bis 4 C-Atomen und Hal Chlor oder Brom bedeuten, hergestellt wird. Der 3-Alkoxy-4-halogen-2E- butensäurealkylester wird darauf erfindungsgemäss entweder über 2 Stufen - mit einem Alkalithioacetat zum 3-Alkoxy-4-thioacetoxy-2E-butensäurealkylester umgesetzt und weiter, nach dessen Isolierung, mit einem Alkalihydroxid zum 4-Alkoxy-2(5H)-thiophenon - umgesetzt oder direkt - mit einem Alkalihydrogensulfid zum 4-Alkoxy-2(5H)-thiophenon - umgewandelt.

Verfährt man nach dem 2-Stufenverfahren, werden die Alkalithioacetate, vorteilhaft unmittelbar vor der Umsetzung, mit dem 3-Alkoxy-4-halogen-2E-butensäurealkylester, zweckmässig durch Umsetzung eines Alkalialkoholates, das seinerseits auf bekannte Weise aus dem jeweiligen Alkalimetall und dem entsprechenden Alkohol hergestellt wurde, mit Thioessigsäure generiert. Bevorzugt wird als Alkalithioacetat das Natriumthioacetat, das entsprechend aus einem Natriumalkoholat, vorzugsweise Natriummethylat und Thioessigsäure, hergestellt wird, verwendet. Die Alkalithioacetatlösung kann dann, zweckmässig bei Temperaturen zwischen 0 und 30°C, mit dem entsprechenden 3-Alkoxy-4-halogen-2E-butensäurealkylester zusammengegeben werden.

Bevorzugte Edukte sind die 3-Alkoxy-4-chlor-2E-butensäuremethylester.

Als Lösungsmittel dient zweckmässig der bei der Generierung des Alkalithioacetats angewendete aliphatische Alkohol.

Bevorzugter aliphatischer Alkohol ist Methanol.

Nach einer Reaktionsdauer von zweckmässig 5 bis 10 Stunden bei Temperaturen zwischen zweckmässig 20 und 50°C kann das abgespaltene Alkalihalogenid abgetrennt werden und der entsprechende 3-Alkoxy-4-thioacetoxy-2E-butensäurealkylester nach üblicher Methode, z.B. durch Eindampfen des Filtrats, gewonnen werden.

Die Ausbeute in dieser Stufe ist praktisch quantitativ.

Die erfindungsgemässen 3-Alkoxy-4-thioacetoxy-2E-butensäurealkylester, bevorzugt die 3-Alkoxy-4-thioacetoxy-2E-butensäuremethylester der Formel

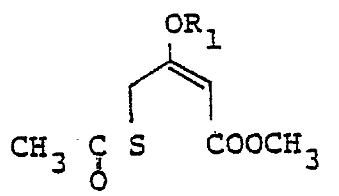

5

worin $R_1$ die genannte Bedeutung hat, sind bisher nicht bekannte Verbindungen. Diese können auf einfache Weise durch Umsetzung mit einem Alkalihydroxid in 4-Alkoxy-2(5H)-thiophenone übergeführt werden.

Als Alkalihydroxid kommt vor allem Natrium- oder Kaliumhydroxid in Frage.

Vorteilhaft wird in Wasser als Lösungsmittel bei Temperaturen von 0 bis 40°C gearbeitet. In der Regel kann bereits nach weniger als 1 Stunde das entsprechende 4-Alkoxy-2(5H)-thiophenon durch Filtration abgetrennt und gegebenenfalls durch Umkristallisation gereinigt werden.

Nach dem Direktverfahren wird zweckmässig so vorgegangen, dass das Alkalihydrogensulfid im Ueberschuss im niederen aliphatischen Alkohol umgesetzt vorgelegt und danach mit dem 4-Alkoxy-3-halogen- 2E-butensäurealkylester umgesetzt wird.

Als Alkalihydrogensulfid wird vorzugsweise das Natriumhydrogensulfid, besonders bevorzugt das Natriumhydrogensulfidmonohydrat, eingesetzt.

Bevorzugte Edukte sind die 3-Alkoxy-4-chlor-2E-butensäuremethylester. Zweckmässig wird auf 1 Mol 3-Alkoxy-4-halogen-2E-butensäurealkylester ein Ueberschuss an Alkalihydrogensulfid von 10 bis 100% veranschlagt.

Als niederer aliphatischer Alkohol wird zweckmässig der dem Esterrest des Eduktes entsprechende Alkohol verwendet. Bevorzugt wird Methanol eingesetzt.

Die Reaktionstemperatur liegt zweckmässig zwischen 20 und 70°C.

Nach einer Reaktionszeit von in der Regel 4 bis 8 Stunden kann auf verfahrensübliche Weise aufgearbeitet und das entsprechende 4-Alkoxy-2(5H)-thiophenon erhalten werden.

Die 4-Alkoxy-2(5H)-thiophenone können als interessante Zwischenprodukte für weitere Thiotetronsäurederivate eingesetzt werden, insbesondere sind sie, darunter vor allem das 4-Methoxy-2(5H)-thiophenon, besonders geeignet zur Herstellung einer hochreinen Thiotetronsäure.

Dazu wird das entsprechende 4-Alkoxy-2(5H)-thiophenon zweckmässig in wasserfreier Essigsäure gelöst. Die Lösung kann dann bei Temperaturen von zweckmässig 20 bis 60°C mit gasförmiger Salzsäure gesättigt werden. Zweckmässig bei der für die Sättigung gewählten Temperatur wird das Reaktionsgemisch vorteilhaft über 15 bis 20 Stunden gerührt.

Nach üblicher Aufarbeitung, zweckmässig durch Entfernen des Lösungsmittels und Waschen des ausgefallenen Produktes, kann ohne zusätzliche Reinigung eine hochreine Thiotetronsäure mit einem Gehalt von grösser als 99% und Ausbeuten grösser als 93% erhalten werden.

Beispiel 1

a) Herstellung von 4-Chlor-3-methoxy-2-E-butensäuremethylester

31,0 g (0,2 Mole) 4-Chloracetessigsäuremethylester wurden mit 106,0 g (1,0 Mol) Orthoameisensäuretrimethylester vermischt. Unter Argon gab man 30,0 g Amberlyst-15-Ionenaustauscherharz unter Rühren hinzu. Unter heftiger Gasentwicklung stieg die Reaktionstemperatur auf 40°C. Nach 5 St unden Rühren liess sich im Dünnschichtchromatogramm kein Edukt mehr nachweisen. Man filtrierte vom Ionenaustauscherharz ab und destillierte den Rückstand im Wasserstrahlvakuum. Das Destillat wurde mit 1,0 g p-Toluolsulfonsäure-monohydrat versetzt und langsam auf 150°C erhitzt, wobei Methanol abdestillierte.

Die Reaktionsmasse wurde anschliessend im Wasserstrahlvakuum destilliert.

Man erhielt 24,7 g einer farblosen Flüssigkeit mit einem Siedepunkt $Kp_{12}$ = 93°C

NMR ($CDCl_3$) δ = 5,16 (s, 1H); 4,67 (s, 2H); 3,73 (s, 6H)

Ausbeute: 75 %.

b) Herstellung von 3-Methoxy-4-thioacetoxy-2E-butensäuremethylester

4,07 g (0,177 Mol) Natrium wurden in 180 ml Methanol gelöst und auf 0°C abgekühlt. Zu dieser Lösung wurden 13,47 g (0,171 Mol) Thioessigsäure getropft. Anschliessend versetzte man diese Lösung bei 0°C mit einer Lösung von 29,15 g (0,150 Mol) 3-Methoxy-4-chlor-2E-butensäuremethylester in 40 ml Methanol. Ueber Nacht liess man bei Raumtemperatur nachrühren. Man fil trierte vom ausgefallenen Salz ab, dampfte das Lösungsmittel am Rotationsverdampfer ab und versetzte zur Fällung des restlichen Salzes mit wenig Methylenchlorid.

Nach Abfiltrieren, Abdampfen des Lösungsmittels und Trocknen des Rückstandes am Hochvakuum erhielt man 36,53 g einer gelbgefärbten Flüssigkeit mit einem Gehalt nach GC von 82,8%.

Dies entsprach 30,25 g 100%iges Produkt ≙ 98,7% Ausbeute.

$Kp_{0,2}$ = 95°C

NMR ($CDCl_3$, 300 MHz) δ = 2,36 (s, 3H), 3,66 (s, 3H),
3,71 (s, 3H), 4,29 (s, 2H),
5,10 (s, 1H)

MS (70 eV)
m/z = 204 ($M^+$, 12), 162 (35), 130 (80), 43 (100)

c) Herstellung von 4-Methoxy-2(5H)-thiophenon

35,77 g (0,145 Mol) 3-Methoxy-4-thioacetoxy-2E-butensäuremethylester (82,8%) wurden vorgelegt und unter Rühren mit einer Lösung von 12,20 g (0,217 Mol) KOH in 45 ml Wasser versetzt. Nach ca. 30 Minuten fiel ein gelblich gefärbter Feststoff aus. Dieses Produkt wurde abgenutscht und nach kurzem Trocknen aus 20 ml Methanol umkristallisiert.

Man erhielt 15,0 g weisses Produkt vom Smp. 90-91°C, (GC: 97,3%).

Dies entsprach einer Ausbeute von 77,4%.

NMR ($CDCl_3$, 300 MHz) δ = 3,87 (s, 3H), 3,91 (s, 2H),
5,49 (s, 1H).

MS (70 eV)
m/z = 130 ($M^+$, 100), 84 (15), 72 (52), 69 (39), 45 (20).

d) Herstellung von Thiotetronsäure

2,60 g (0,0194 Mol) 4-Methoxy-2(5H)-thiophenon (97,3%) wurden in 30 ml Essigsäure gelöst und bei 40°C mit gasförmiger Salzsäure gesättigt. Man rührte die Reaktionsmischung bei dieser Temperatur 16 Stunden lang. Anschliessend wurde die Essigsäure am Rotationsverdampfer unter Vakuum eingeengt. Das Rohprodukt wurde mit 10 ml Toluol gewaschen, abgenutscht und am Hochvakuum getrocknet.

Man erhielt 2,13 g nahezu weisse, kristalline Thiotetronsäure vom Smp. 120°C mit einem Gehalt (NaOH-Titr.) von 99,5%.

Dies entsprach 2,12 g 100%igem Produkt = 93,9% Ausbeute.

Beispiel 2

Herstellung von 4-Methoxy-2(5H)-thiophenon aus 4-Chlor-3-methoxy-2E-butensäuremethylester und Natriumhydrogensulfid

11,6 g (0,14 Mol) Natriumhydrogensulfidmonohydrat 90%ig wurden in 90 ml Methanol gelöst. Zu dieser Lösung gab man bei 50°C innerhalb von 4 Stunden tropfenweise eine Lösung von 17,0 g (0,1 Mol) 4-Chlor-3-methoxy-2E-butensäuremethylester 96,7% in 10 ml Methanol. Man liess 2 Stunden nachrühren und destillierte anschliessend das Methanol am Rotationverdampfer unter Vakuum ab. Der Rückstand wurde mit 100 ml Wasser versetzt und zweimal mit je 80 ml Methylenchlorid extrahiert. Die organische Phase wurde über Natriumsulfat getrocknet und eingedampft. Der Rückstand wurde aus 15 ml Ethanol heiss umkristallisiert. Man erhielt 5,12 g gelbgefärbtes Produkt vom Smp. 90°C.
Gehalt (GC): 96%.
Ausbeute: 37,8%.

**Patentansprüche für die Vertragstaaten AT, BE, CH, DE, FR, GB, IT, LI, LU, NL, SE**

1. Verfahren zur Herstellung von 4-Alkoxy-2(5H)-thiophenonen, der Formel

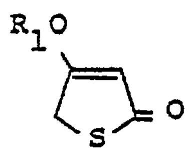

1

worin $R_1$ Alkyl geradkettig oder verzweigt, mit 1 bis 4 C-Atomen bedeutet, dadurch gekennzeichnet, dass ein 3-Alkoxy-4-halogen-2E-butensäurealkylester der Formel

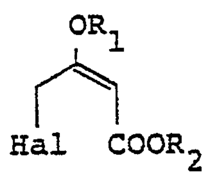

2

worin $R_1$ und $R_2$ Alkyl geradkettig oder verzweigt, mit 1 bis 4 C-Atomen, und Hal Chlor oder Brom bedeuten, entweder mit einem Alkalisalz der Thioessigsäure zu einem 3-Alkoxy-4- thioacetoxy-2E-butensäurealkylester umgesetzt, dieser isoliert und weiter mit einem Alkalihydroxid zum Endprodukt übergeführt wird, oder direkt mit einem Alkalihydrogensulfid zum Endprodukt umgesetzt wird.

2. Verfahren nach Patentanspruch 1, dadurch gekennzeichnet, dass der 3-Alkoxy-4-halogen-2E-butensäurealkylester auf bekannte Weise, ausgehend von einem 4-Halogenacetessigsäurealkylester, durch Umsetzung mit Orthoameisensäuretrialkylester hergestellt wird.

3. Verfahren nach Patentanspruch 1 oder 2, dadurch gekennzeichnet, dass als Alkalisalz der Thioessigsäure Natriumthioacetat eingesetzt wird.

4. Verfahren nach Patentansprüchen 1, 2 oder 3, dadurch gekennzeichnet, dass als Alkalihydrogensulfid Natriumhydrogensulfid eingesetzt wird.

5. Verfahren nach Patentansprüchen 1, 2, 3 oder 4, dadurch gekennzeichnet, dass die Umsetzung mit dem Alkalisalz der Thioessigsäure oder die Umsetzung mit dem Alkalihydrogensulfid in Gegenwart eines niederen aliphatischen Alkohols als Lösungsmittel erfolgt.

6. Verfahren nach Patentansprüchen 1, 2, 3, 4 oder 5, dadurch gekennzeichnet, dass die Umsetzung mit dem Alkalisalz der Thioessigsäure bei Temperaturen zwischen 20 und 60°C erfolgt.

7. Verfahren nach Patentansprüchen 1, 2, 3, 4, 5 oder 6, dadurch gekennzeichnet, dass die Umsetzung mit dem Alkalihydroxid in Gegenwart von Wasser erfolgt.

8. Verfahren nach Patentansprüchen 1, 2, 3, 4, 5, 6 oder 7, dadurch gekennzeichnet, dass die direkte Umsetzung mit dem Alkalihydrogensulfid bei Temperaturen zwischen 20 bis 70°C erfolgt.

9. Verwendung der gemäss dem Verfahren nach Patentanspruch 1 hergestellten 4-Alkoxy-2(5H)-thiophenone zur Herstellung einer hochreinen Thiotetronsäure, gekennzeichnet durch Umsetzung der 4-Alkoxy-2(5H)-thiophenone mit gasförmiger Salzsäure in wasserfreier Essigsäure.

10. 3-Alkoxy-4-thioacetoxy-2E-butensäurealkylester der Formel

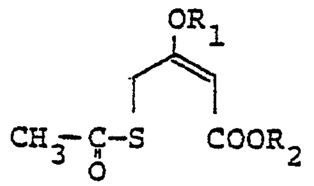 3

worin $R_1$ und $R_2$ die genannte Bedeutung haben.

11. 3-Methoxy-4-thioacetoxy-2E-butensäuremethylester der Formel

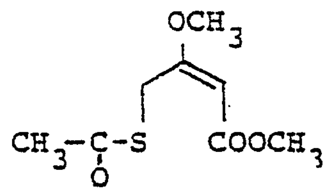 4

**Patentansprüche für den Vertragstaat ES**

1. Verfahren zur Herstellung von 4-Alkoxy-2(5H)-thiophenonen, der Formel

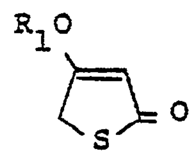 1

worin $R_1$ Alkyl geradkettig oder verzweigt, mit 1 bis 4 C-Atomen bedeutet, dadurch gekennzeichnet, dass ein 3-Alkoxy-4-halogen-2E-butensäurealkylester der Formel

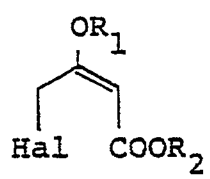 2

worin $R_1$ und $R_2$ Alkyl geradkettig oder verzweigt, mit 1 bis 4 C-Atomen, und Hal Chlor oder Brom bedeuten, entweder mit einem Alkalisalz der Thioessigsäure zu einem 3-Alkoxy-4-thioacetoxy-2E-butensäurealkylester umgesetzt, dieser isoliert und weiter mit einem Alkalihydroxis zum Endprodukt übergeführt wird, oder direkt mit einem Alkalihydrogensulfid zum Endprodukt umgesetzt wird.

2. Verfahren nach Patentanspruch 1, dadurch gekennzeichnet, dass der 3-Alkoxy-4-halogen-2E-butensäurealkylester auf bekannte Weise, ausgehend von einem 4-Halogenacetessigsäurealkylester, durch Umsetzung mit Orthoameisensäuretrialkylester hergestellt wird.

3. Verfahren nach Patentanspruch 1 oder 2, dadurch gekennzeichnet, dass als Alkalisalz der Thioessigsäure Natriumthioacetat eingesetzt wird.

4. Verfahren nach Patentansprüchen 1, 2 oder 3, dadurch gekennzeichnet, dass als Alkalihydrogensulfid Natriumhydrogensulfid eingesetzt wird.

5. Verfahren nach Patentansprüchen 1, 2, 3 oder 4, dadurch gekennzeichnet, dass die Umsetzung mit dem Alkalisalz der Thioessigsäure oder die Umsetzung mit dem Alkalihydrogensulfid in Gegenwart eines niederen aliphatischen Alkohols als Lösungsmittel erfolgt.

6. Verfahren nach Patentansprüchen 1, 2, 3, 4 oder 5, dadurch gekennzeichnet, dass die Umsetzung mit dem Alkalisalz der Thioessigsäure bei Temperaturen zwischen 20 und 60°C erfolgt.

7. Verfahren nach Patentansprüchen 1, 2, 3, 4, 5 oder 6, dadurch gekennzeichnet, dass die Umsetzung mit dem Alkalihydroxid in Gegenwart von Wasser erfolgt.

8. Verfahren nach Patentansprüchen 1, 2, 3, 4, 5, 6 oder 7, dadurch gekennzeichnet, dass die direkte Umsetzung mit dem Alkalihydrogensulfid bei Temperaturen zwischen 20 bis 70°C erfolgt.

9. Verfahren zur Herstellung hochreiner Thiotetronsäure, dadurch gekennzeichnet, daß die gemäß dem Verfahren nach Patentanspruch 1 hergestellten 4-Alkoxy-2(5H)-thiophenone in wasserfreier Essigsäure mit gasförmiger Salzsäure umgesetzt werden.

**Claims for the Contracting States BE, CH, DE, AT, FR, GB, IT, LI, LU, NL, SE**

1. Process for the preparation of 4-alkoxy-2(5H)-thiophenones of the formula:

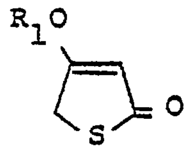

1

wherein $R_1$ is a straight or branched-chain alkyl group having 1 to 4 carbon atoms, characterized in that a 3-alkoxy-4-halo-2E-butenoic acid alkyl ester of the formula

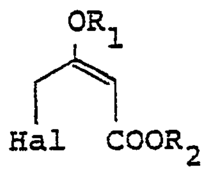

2

wherein $R_1$ and $R_2$ each are a straight or branched-chain alkyl group having 1 to 4 carbon atoms and Hal is chlorine or bromine, is either reacted with an alkali salt of thioacetic acid into a 3-alkoxy-4-thioacetoxy-2E-butenoic acid alkyl ester, the latter is isolated and further transformed into the end product by means of an alkali hydroxide, or directly reacted to the end product with an alkali hydrogen sulfide.

2. Process according to patent claim 1, characterized in that the 3-alkoxy-4-halo-2E-butenoic acid alkyl ester is prepared in known manner by reaction with orthoformic acid trialkyl ester starting with a 4-haloacetoacetic acid alkyl ester.

3. Process according to patent claim 1 or 2, characterized in that sodium thioacetate is used as alkali salt of thioacetic acid.

4. Process according to patent claims 1, 2 or 3, characterized in that sodium hydrogen sulfide is used as alkali hydrogen sulfide.

5. Process according to patent claims 1, 2, 3 or 4, characterized in that the reaction with the alkali salt of thioacetic acid or the reaction with alkali hydrogen sulfide is carried out in the presence of a low aliphatic alcohol as solvent.

6. Process according to patent claims 1, 2, 3, 4 or 5, characterized in that the reaction with the alkali salt of thioacetic acid is carried out at temperatures between 20 and 60°C.

7. Process according to patent claims 1, 2, 3, 4, 5 or 6, characterized in that the reaction with alkali hydroxide is carried out in the presence of water.

8. Process according to patent claims 1, 2, 3, 4, 5, 6 or 7, characterized in that the direct reaction with alkali hydrogen sulfide is carried out at temperatures between 20 and 70°C.

9. The use of the 4-alkoxy-2(5H)-thiophenones prepared according to the process of patent claim 1 in the preparation of thiotetronic acid of high purity, characterized by the reaction of the 4-alkoxy-2(5H)-thiophenones with gaseous hydrochloric acid in anhydrous acetic acid.

10. 3-Alkoxy-4-thioacetoxy-2E-butenoic acid alkyl esters of the formula

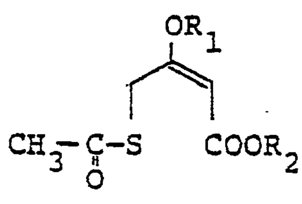

3

wherein $R_1$ and $R_2$ have the indicated meanings.

11. 3-Methoxy-4-thioacetoxy-2E-butenoic acid methyl ester of the formula

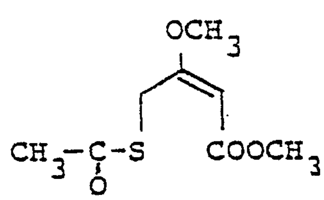

4

**Claims for the Contracting State ES**

1. Process for the preparation of 4-alkoxy-2(5H)-thiophenones of the formula:

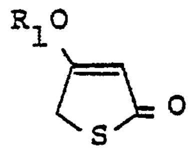

1

wherein $R_1$ is a straight or branched-chain alkyl group having 1 to 4 carbon atoms, characterized in that a 3-alkoxy-4-halo-2E-butenoic acid alkyl ester of the formula

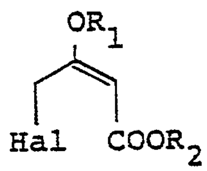

2

wherein $R_1$ and $R_2$ each are a straight or branched-chain alkyl group having 1 to 4 carbon atoms and Hal is chlorine or bromine, is either reacted with an alkali salt of thioacetic acid into a 3-alkoxy-4-thioacetoxy-2E-butenoic acid alkyl ester, the latter is isolated and further transformed into the end product by means of an alkali hydroxide, or directly reacted to the end product with an alkali hydrogen sulfide.

2. Process according to patent claim 1, characterized in that the 3-alkoxy-4-halo-2E-butenoic acid alkyl ester is prepared in known manner by reaction with orthoformic acid trialkyl ester starting with a 4-haloacetoacetic acid alkyl ester.

3. Process according to patent claim 1 or 2, characterized in that sodium thioacetate is used as alkali salt of thioacetic acid.

4. Process according to patent claims 1, 2 or 3, characterized in that sodium hydrogen sulfide is used as alkali hydrogen sulfide.

5. Process according to patent claims 1, 2 3 or 4, characterized in that the reaction with the alkali salt of thioacetic acid or the reaction with alkali hydrogen sulfide is carried out in the presence of a low aliphatic alcohol as solvent.

6. Process according to patent claims 1, 2, 3, 4 or 5, characterized in that the reaction with the alkali salt of thioacetic acid is carried out at temperatures between 20 and 60°C.

7. Process according to patent claims 1, 2, 3, 4, 5 or 6, characterized in that the reaction with alkali hydroxide is carried out in the presence of water.

8. Process according to patent claims 1, 2, 3, 4, 5, 6 or 7, characterized in that the direct reaction with alkali hydrogen sulfide is carried out at temperatures between 20 and 70°C.

9. Process for the preparation of thiotetronic acid of high purity, characterized in that the 4-alkoxy-2(5H)-thiophenones prepared according to the process of patent claim 1 are reacted in anhydrous acetic acid with gaseous hydrochloric acid.

**Revendications pour les Etats contractants BE, CH, DE, AT, FR, GB, IT, LI, LU, NL, SE**

1. Procédé de préparation de 4-alcoxy-2(5H)-thiophénones répondant à la formule

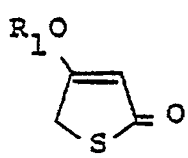

1

dans laquelle $R_1$ désigne un groupe alkyle linéaire ou ramifié en $C_1$ à $C_4$, caractérisé en ce qu'on fait réagir un 3-alcoxy-4-halogéno-2E-butènoaté d'alkyle répondant à la formule

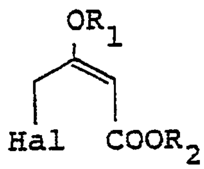

2

dans laquelle $R_1$ et $R_2$ désignent un groupe alkyle linéaire ou ramifié, en $C_1$ à $C_4$, et Hal un atome de chlore ou de brome, soit avec un sel alcalin de l'acide thioacétique pour donner un 3-alcoxy-4-thioacétoxy-2E-butènoate d'alkyle, en ce qu'on isole celui-ci, puis en ce qu'on le transforme en le produit final avec un hydroxyde alcalin, soit directement avec un hydrogénosulfure alcalin pour donner le produit final.

2. Procédé suivant la revendication 1, caractérisé en ce qu'on prépare le 3-alcoxy-4-halogéno-2E-butènoate d'alkyle d'une manière connue, en partant d'un 4-halogénoacétoacétate d'alkyle, par réaction avec un orthoformiate de trialkyle.

3. Procédé suivant les revendications 1 ou 2, caractérisé en ce qu'on utilise comme sel alcalin de l'acide thioacétique le thioacétate de sodium.

4. Procédé suivant les revendications 1, 2 ou 3, caractérisé en ce qu'on utilise comme hydrogénosulfure alcalin l'hydrogénosulfure de sodium.

5. Procédé suivant les revendications 1, 2, 3 ou 4, caractérisé en ce qu'on effectue la réaction avec le sel alcalin de l'acide thioacétique ou la réaction avec l'hydrogénosulfure alcalin en présence d'un alcool aliphatique inférieur comme solvant.

6. Procédé suivant les revendications 1, 2, 3, 4 ou 5, caractérisé en ce qu'on effectue la réaction avec le sel alcalin de l'acide thioacétique à des températures comprises entre 20 et 60°C.

7. Procédé suivant les revendications 1, 2, 3, 4, 5 ou 6, caractérisé en ce qu'on effectue la réaction avec l'hydroxyde alcalin en présence d'eau.

8. Procédé suivant les revendications 1, 2, 3, 4, 5, 6 ou 7, caractérisé en ce qu'on effectue la réaction directe avec l'hydrogénosulfure alcalin à des températures comprises entre 20 et 70°C.

9. Utilisation des 4-alcoxy-2(5H)-thiopénones préparées conformément au procédé selon la revendication 1, pour la préparation d'un acide thiotétronique de haute pureté, caractérisée par la réaction des 4-alcoxy-2(5H)-thiophénones avec de l'acide chlorhydrique gazeux dans de l'acide acétique anhydre.

10. 3-alcoxy-4-thioacétoxy-2E-butènoate d'alkyle répondant à la formule

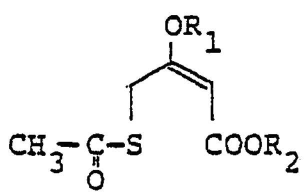

3

dans laquelle $R_1$ et $R_2$ ont la signification indiquée.

11. 3-méthoxy-4-thioacétoxy-2E-butènoate de méthyle répondant à la formule

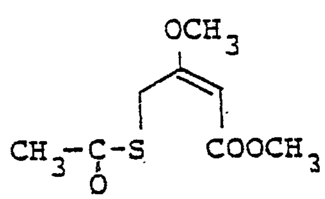

4

**Revendications pour l'Etat contractant ES**

1. Procédé de préparation de 4-alcoxy-2(5H)-thiophénones répondant à la formule

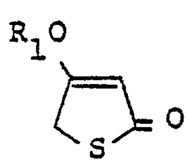

1

dans laquelle $R_1$ désigne un groupe alkyle linéaire ou ramifié en $C_1$ à $C_4$, caractérisé en ce qu'on fait réagir un 3-alcoxy-4-halogéno-2E-butènoate d'alkyle répondant à la formule

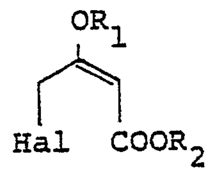

2

dans laquelle $R_1$ et $R_2$ désignent un groupe alkyle linéaire ou ramifié, en $C_1$ à $C_4$, et Hal un atome de chlore ou de brome, soit avec un sel alcalin de l'acide thioacétique pour donner un 3-alcoxy-4-thioacétoxy-2E-butènoate d'alkyle, en ce qu'on isole celui-ci, puis en ce qu'on le transforme en le produit final avec un hydroxyde alcalin, soit directement avec un hydrogénosulfure alcalin pour donner le produit final.

2. Procédé suivant la revendication 1, caractérisé en ce que le 3-alcoxy-4-halogéno-2E-butènoate d'alkyle est préparé d'une manière connue, en partant d'un 4-halogénoacétoacétate d'alkyle, par réaction avec un orthoformiate de trialkyle.

3. Procédé suivant les revendications 1 ou 2, caractérisé en ce qu'on utilise comme sel alcalin de l'acide thioacétique le thioacétate de sodium.

4. Procédé suivant les revendications 1, 2 ou 3, caractérisé en ce qu'on utilise comme hydrogénosulfure alcalin l'hydrogénosulfure de sodium.

5. Procédé suivant les revendications 1, 2, 3 ou 4, caractérisé en ce qu'on effectue la réaction avec le sel alcalin de l'acide thioacétique ou la réaction avec l'hydrogénosulfure alcalin en présence d'un alcool aliphatique inférieur comme solvant.

6. Procédé suivant les revendications 1, 2, 3, 4 ou 5, caractérisé en ce qu'on effectue la réaction avec le sel alcalin de l'acide thioacétique à des températures comprises entre 20 et 60°C.

7. Procédé suivant les revendications 1, 2, 3, 4, 5 ou 6, caractérisé en ce qu'on effectue la réaction avec l'hydroxyde alcalin en présence d'eau.

8. Procédé suivant les revendications 1, 2, 3, 4, 5, 6 ou 7, caractérisé en ce qu'on effectue la réaction directe avec l'hydrogénosulfure alcalin à des températures comprises entre 20 et 70°C.

9. Procédé de préparation d'un acide thiotétronique de haute pureté, caractérisé en ce qu'on fait réagir des 4-alcoxy-2(5H)-thiophénones préparées conformément au procédé selon la revendication 1 avec de l'acide chlorhydrique gazeux, dans de l'acide acétique anhydre.